# EUROPÄISCHE PATENTSCHRIFT

(11) **EP 2 401 315 B1**
(45) Veröffentlichungstag und Bekanntmachung des Hinweises auf die Patenterteilung: **22.08.2018**
(21) Anmeldenummer: 10702442.4
(22) Anmeldetag: 01.02.2010
(51) Int. Cl.: C08G 61/12, C09D 125/00, C09D 165/00

(54) **VERNETZBARE UND VERNETZTE POLYMERE, VERFAHREN ZU DEREN HERSTELLUNG SOWIE DEREN VERWENDUNG**
CROSS-LINKABLE AND CROSS-LINKED POLYMERS, METHOD FOR THE PRODUCTION THEREOF, AND USE THEREOF
POLYMÈRES RÉTICULABLES ET RÉTICULÉS, LEURS PROCÉDÉS DE PRÉPARATION ET LEUR UTILISATION

(30) Priorität: 27.02.2009 DE 102009010714
(43) Veröffentlichungstag der Anmeldung: 04.01.2012
(73) Patentinhaber: Merck Patent GmbH, 64293 Darmstadt (DE)
(72) Erfinder: MEYER, Frank-Egon, Winchester Hampshire SO23 8AZ (GB); SCHULTE, Niels, 65779 Kelkheim (DE); SCHEURICH, René, Peter, 64846 Gross-Zimmern (DE); ANÉMIAN, Rémi, Manouk, Seoul 657-169 (KP); LUDEMANN, Aurélie, 60322 Frankfurt am Main (DE); JULLIART, Alice, 60311 Frankfurt/Main (DE)
(86) Internationale Anmeldenummer: PCT/EP2010/000591
(87) Internationale Veröffentlichungsnummer: WO 2010/097156

(56) Entgegenhaltungen:
- EP-A1- 1 832 616
- WO-A1-2006/043087
- US-A1- 2001 017 155
- DATABASE CA [Online] CHEMICAL ABSTRACTS SERVICE, COLUMBUS, OHIO, US; 18. Juli 2003 (2003-07-18), NOGUCHI, TAKANOBU ET AL: "Process for production of high-molecular compounds useful for polymer LED or the like" XP002574884 gefunden im STN Database accession no. 2003:551570 -& WO 03/057762 A1 (SUMITOMO CHEMICAL COMPANY, LIMITED, JAPAN) 17. Juli 2003 (2003-07-17)

## Beschreibung

Die vorliegende Erfindung betrifft vernetzbare und vernetzte Polymere. Die Erfindung ist ferner auf die Verwendung dieser Polymere in elektronischen Vorrichtungen sowie auf die entsprechenden elektronischen Vorrichtungen selbst gerichtet.

Elektronische Vorrichtungen, welche organische, metallorganische und/oder polymere Halbleiter enthalten, werden immer häufiger in kommerziellen Produkten verwendet oder stehen kurz vor der Markteinführung. Als Beispiele seien hier Ladungstransportmaterialien auf organischer Basis (z. B. Lochtransporter auf Triarylamin-Basis) in Kopiergeräten, organischen oder polymeren Leuchtdioden (OLEDs oder PLEDs) und in Anzeige- und Displayvorrichtungen oder organische Photorezeptoren in Kopierern genannt. Organische Solarzellen (O-SC), organische Feldeffekt-Transistoren (O-FET), organische Dünnfilm-Transistoren (O-TFT), organische Schaltelemente (O-IC), organische optische Verstärker und organische Laserdioden (O-Laser) sind in einem fortgeschrittenen Entwicklungsstand und können in der Zukunft große Bedeutung erlangen.

Viele dieser elektronischen Vorrichtungen weisen unabhängig von dem jeweiligen Verwendungszweck folgenden allgemeinen Schichtaufbau auf, der für die jeweilige Anwendung angepasst werden kann:
(1) Substrat,
(2) Elektrode, häufig metallisch oder anorganisch, aber auch aus organischen bzw. polymeren leitfähigen Materialien,
(3) Ladungsinjektionsschicht/en bzw. Zwischenschicht/en, beispielsweise zum Ausgleich von Unebenheiten der Elektrode ("planarisation layer"), häufig aus einem leitfähigen, dotierten Polymer,
(4) Organische Halbleiter,
(5) evtl. weitere Ladungstransport-, Ladungsinjektions- bzw. Ladungsblockierschichten,
(6) Gegenelektrode, Materialien wie unter (2) genannt,
(7) Verkapselung.

Die obige Anordnung stellt den allgemeinen Aufbau einer organischen, elektronischen Vorrichtung dar, wobei verschiedene Schichten zusammengefasst werden können, so dass im einfachsten Fall eine Anordnung aus zwei Elektroden resultiert, zwischen denen sich eine organische Schicht befindet. Die organische Schicht erfüllt in diesem Fall alle Funktionen, einschließlich der Emission von Licht im Fall von OLEDs. Ein derartiges System ist beispielsweise in der WO 90/13148 A1 auf der Basis von Poly-(p-phenylenen) beschrieben.

Ein Problem, das sich in einem derartigen "Dreischichtsystem" ergibt, ist jedoch die fehlende Steuerung der Ladungstrennung oder die fehlende Möglichkeit, die einzelnen Bestandteile in unterschiedlichen Schichten bezüglich ihrer Eigenschaften zu optimieren, wie es beispielsweise bei SMOLEDs ("small-molecule OLEDs") durch einen mehrschichtigen Aufbau einfach gelöst ist.

Eine "small molecule OLED" besteht beispielsweise aus einer oder mehreren organischen Lochinjektionsschichten, Lochtransportschichten, Emissionsschichten, Elektronentransportschichten und/oder Elektroneninjektionsschichten sowie einer Anode und einer Kathode, wobei sich das ganze System gewöhnlich auf einem Glassubstrat befindet. Der Vorteil einer solchen Mehrlagenstruktur besteht darin, dass verschiedene Funktionen der Ladungsinjektion, des Ladungstransports und der Emission in die verschiedenen Schichten aufgeteilt und somit die Eigen-schaften der jeweiligen Schichten separat modifiziert werden können.

Das Aufbringen der Schichten in SMOLEDs erfolgt gewöhnlich durch Aufdampfen in einer Vakuumkammer. Dieses Verfahren ist jedoch aufwändig und somit teuer und insbesondere für große Moleküle, wie beispielsweise Polymere, ungeeignet.

Polymere OLED-Materialien werden deshalb meist durch Beschichtung aus Lösung aufgetragen. Die Herstellung einer mehrschichtigen, organischen Struktur durch Beschichtung aus Lösung erfordert jedoch, dass das Lösungsmittel der aufzubringenden Schicht die jeweils vorhergehende Schicht nicht wieder anlöst, quellt oder gar zerstört. Die Wahl des Lösungsmittels erweist sich jedoch als schwierig, da die eingesetzten, organischen Polymere gewöhnlich ähnliche chemische Strukturen sowie Eigenschaften, insbesondere ähnliche Lösungseigenschaften, besitzen.

Entsprechend sind polymere OLEDs (PLEDs) gemäß dem Stand der Technik gewöhnlich nur aus einer einschichtigen oder höchstens zweischichtigen, organischen Struktur aufgebaut, wobei beispielsweise eine der Schichten für die Lochinjektion und den Lochtransport verwendet wird und die zweite Schicht beispielsweise für die Injektion und den Transport von Elektronen sowie für die Emission verwendet wird.

Vorteilhaft bei den polymeren OLEDs wäre jedoch auch eine Mehrschichtstruktur wie bei den SMOLEDs. Hierzu werden im Stand der Technik verschiedene Ansätze beschrieben.

So offenbart beispielsweise die EP 0 637 899 A1 eine elektrolumineszierende Anordnung, enthaltend eine oder mehrere organische Schichten, wobei eine oder mehrere der Schichten durch thermische oder strahlungsindizierte Vernetzung erhalten werden.

Bei der strahlungsinduzierten Vernetzung mit energiereicher, elektromagnetischer Strahlung sind häufig Moleküle oder Molekülteile notwendig, die eine radikalische, kationische oder anionische Polymerisation auslösen können. Es ist im Stand der Technik jedoch bekannt, dass solche Moleküle oder Molekülteile negative Einflüsse auf die Funktion einer optoelektronischen Vorrichtung haben können. Auch kann die Verwendung der energiereichen, elektromagnetischen Strahlung problematisch sein.

Ein Problem bei der thermischen Vernetzung ist, dass die polymeren Schichten einer relativ hohen Temperatur ausgesetzt werden, was zum Teil wieder zur Zerstörung der entsprechenden Schicht oder zur Bildung unerwünschter Nebenprodukte führt.

So offenbart beispielsweise die WO 96/20253 ein lumineszierendes, filmbildendes, durch Lösungsmittelbehandlung herstellbares, vernetztes Polymer, wobei Azidgruppen, die an der Polymerhauptkette hängen, thermisch vernetzt werden.

Die US 6,107,452 offenbart ein Verfahren zur Bildung einer mehrlagigen Vorrichtung, bei dem Oligomere mit endständigen Vinylgruppen aus Lösung abgeschieden und zu unlöslichen Polymeren vernetzt werden, auf denen weitere Schichten abgeschieden werden können.

K. Meerholz et al. (Nature, Band 421, 20. Februar 2003, Seiten 829 bis 832) offenbaren die Herstellung einer mehrschichtigen, organischen, Licht emittierenden Vorrichtung, wobei eine Vernetzung durch Einbau Oxetanfunktionalisierter Spirobifluoren-Grundeinheiten in das Polymer erreicht wird.

Die WO 2006/043087 offenbart, dass mit Vernetzungsgruppen funktionalisierte Fluorene nicht sehr wirksam vernetzen. Das bedeutet, dass das Polymer selbst nach dem Vernetzen teilweise löslich bleiben kann. Demzufolge wird die Integrität der Schicht bei Aufbringung einer nachfolgenden Schicht evtl. nicht bewahrt.

Es besteht somit weiterhin ein Bedarf nach Polymeren, die eine zur Vernetzung geeignete funktionelle Gruppe aufweisen, sich leicht, d.h. mit geringem Energieaufwand, vernetzen lassen und keine negativen Auswirkungen auf die Funktion einer elektronischen Vorrichtung haben.

Die Aufgabe der vorliegenden Erfindung bestand somit in der Bereitstellung solcher Polymere.

Überraschenderweise wurde gefunden, dass para-substituierte Aryldiarylamine, bei denen die 4-Position eines Arylrings mit einer vernetzbaren Gruppe versehen ist, oder Homologe davon, die entweder direkt über eine Arylgruppe oder einen nicht aromatischen Spacer an das Polymergrundgerüst, wie beispielsweise Indenofluoren, gebunden werden, diese Nachteile nicht zeigen. Diese Struktureinheit übt keinerlei Einfluss auf die opto-elektronischen Eigenschaften der Polymerhauptkette aus, ermöglicht jedoch eine Vernetzung zwischen zwei oder mehr Hauptketten.

Dies ist deshalb überraschend, da, wie oben bereits angesprochen, die WO 2006/043087 offenbart, dass insbesondere mit Vernetzungsgruppen funktionalisierte Fluorene nicht sehr wirksam vernetzen. Die Vernetzung kann dabei thermisch oder strahlungsinduziert erfolgen, wobei aufgrund des guten Vernetzungsverhaltens des erfindungsgemäßen Systems geringere Energiemengen erforderlich sind als bei den bekannten Systemen. Dadurch kommt es zu weniger unerwünschten Nebenprodukten bei der Vernetzung. Das erfindungsgemäße Polymer weist zudem eine sehr hohe Stabilität auf.

Die Erfindung stellt dazu ein Polymer bereit, das 0,1 bis 50 mol% mindestens einer Struktureinheit der allgemeinen Formel I, 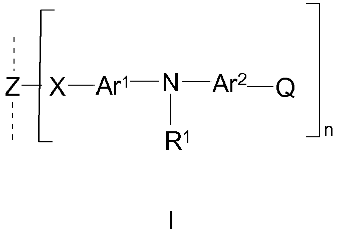 enthält, wobei
- Z: ein aromatisches oder heteroaromatisches Ringsystem mit 5 bis 60 Ringatomen ist, welches mit einem oder mehreren Resten R substituiert sein kann,
- X: ausgewählt ist aus einer direkten Bindung, einer linearen oder verzweigten Alkylen-Gruppe mit 1 bis 20 C-Atomen, in der eine oder mehrere nicht benachbarte CH₂-Gruppen durch -O-, -S-, -NH-, -N(CH₃)-, -N-CO-, -N-CO-O-, -N-CO-N, -CO-, -O-CO-, -S-CO-, -O-COO-, -CO-S-, -CO-O-, -CH(Halogen)-, -CH(CN)-, -CH=CH- oder -C≡C- ersetzt sein können, oder einer cyclischen Alkylgruppe,
- Ar¹, Ar²: jeweils unabhängig voneinander eine Aryl- oder Heteroarylgruppe mit 5 bis 60 Ringatomen ist, welche mit einem oder mehreren beliebigen Resten R substituiert oder miteinander verknüpft sein können, wobei Ar¹ eine 1,4-verknüpfte Aryl- oder Heteroarylgruppe ist,
- Q: eine vernetzbare Gruppe ist, ausgewählt aus Vinyl- oder Alkenylgruppe,
- R: bei jedem Auftreten unabhängig voneinander ausgewählt ist aus F, Cl, Br, I, N(Ar)₂, N(R²)₂, CN, NO₂, Si(R²)₃, B(OR²)₂, C(=O)Ar, C(=O)R², P(=O)(Ar)₂, P(=O)(R²)₂, S(=O)Ar, S(=O)R², S(=O)₂Ar, S(=O)₂R², -CR²=CR²Ar, OSO₂R², einer geradkettigen Alkyl-, Alkoxy- oder Thioalkoxygruppe mit 1 bis 40 C-Atomen oder einer verzweigten oder cyclischen Alkyl-, Alkoxy- oder Thio-alkoxygruppe mit 3 bis 40 C-Atomen, die jeweils mit einem oder mehreren Resten R² substituiert sein kann, wobei eine oder mehrere nicht benachbarte CH₂-Gruppen durch R²C=CR², C≡C, Si(R²)₂, Ge(R²)₂, Sn(R²)₂, C=O, C=S, C=Se, C=NR², P(=O)(R²), SO, SO₂, NR², O, S oder CONR² ersetzt sein können und wobei ein oder mehrere H-Atome durch F, Cl, Br, I, CN oder NO₂ ersetzt sein können, oder einem aromatischen oder heteroaromatischen Ringsystem mit 5 bis 60 Ringatomen, das jeweils durch einen oder mehrere Reste R² substituiert sein kann, oder einer Aryloxy- oder Heteroaryloxygruppe mit 5 bis 60 Ring-atomen, die durch einen oder mehrere Reste R² substituiert sein kann, oder einer Kombination dieser Systeme, wobei zwei oder mehrere Substituenten R auch miteinander ein mono- oder polycyclisches, aliphatisches oder aromatisches Ringsystem bilden können, wobei R² jeweils unabhängig voneinander H oder ein aliphatischer oder aromatischer Kohlenwasserstoffrest mit 1 bis 20 C-Atomen ist,
- R¹: eine geradkettige Alkyl-, Alkoxy- oder Thioalkoxygruppe mit 1 bis 40 C-Atomen oder eine verzweigte oder cyclische Alkyl-, Alkoxy- oder Thioalkoxygruppe mit 3 bis 40 C-Atomen ist oder eine Silylgruppe oder eine substituierte Ketogruppe mit 1 bis 40 C-Atomen, oder ein substituiertes oder unsubstituiertes aromatisches oder heteroaromatisches Ringsystem mit 5 bis 60 Ringatomen, oder eine Aryloxy- oder Heteroaryloxygruppe mit 5 bis 60 Ringatomen, oder eine Kombination dieser Systeme, oder Ar² ist, wobei ein oder mehrere H-Atome durch Fluor ersetzt sein können,
- n: 1, 2, 3 oder 4 ist, und
die gestrichelten Linien die Verknüpfung im Polymer darstellen,
wobei gilt, wenn n = 1 ist,
dass X keine direkte Bindung ist.

"Vernetzbare Gruppe" bedeutet eine funktionelle Gruppe, die in der Lage ist, irreversibel zu reagieren. Dadurch wird ein vernetztes Material gebildet, das unlöslich ist. Die Vernetzung kann gewöhnlich durch Wärme oder durch UV-, Mikrowellen-, Röntgen- oder Elektronenstrahlung unterstützt werden. Durch die hohe Stabilität des erfindungsgemäßen Polymers kommt es bei der Vernetzung zu weniger Nebenproduktbildung. Zudem vernetzen die vernetzbaren Gruppen im erfindungsgemäßen Polymer sehr leicht, so dass geringere Energiemengen für die Vernetzung erforderlich sind (z.B. < 200°C bei der thermischen Vernetzung).

X dient als sogenannter Spacer oder Abstandsgruppe. Im Sinne dieser Erfindung kann X jedoch auch eine direkte Bindung darstellen, wobei dann Ar¹ direkt an Z gebunden ist.

X ist eine lineare oder verzweigte Alkylen-Gruppe mit 1 bis 20 C-Atomen, besonders bevorzugt mit 1 bis 12 C-Atomen, in der eine oder mehrere nicht benachbarte CH₂-Gruppen durch -O-, -S-, -NH-, -N(CH₃)-, -N-CO-, - N-CO-O-, -N-CO-N, -CO-, -O-CO-, -S-CO-, -O-COO-, -CO-S-, -CO-O-, -CH(Halogen)-, -CH(CN)-, -CH=CH- oder -C≡C- ersetzt sein können, oder eine cyclische Alkylgruppe, bevorzugt Cyclohexan oder ein Cyclohexanderivat mit 1,4- oder 1,3-Verknüpfung. Weitere mögliche Abstandsgruppen X sind zum Beispiel -(CH₂)ₒ-, -(CH₂CH₂O)ₚ-CH₂CH₂-, -CH₂CH₂-S-CH₂CH₂- oder -CH2CH2-NH-CH2CH2-, mit o = 2 bis 12 und p = 1 bis 3 aber auch -O-.

Bevorzugte Abstandsgruppen X sind Ethylen, Propylen, Butylen, Pentylen, Hexylen, Heptylen, Octylen, Nonylen, Decylen, Undecylen, Dodecylen, Octadecylen, Ethylenoxyethylen, Methylenoxybutylen, Ethylenthioethylen, Ethylen-N-methyl-iminoethylen, 1-Methylalkylen, Ethenylen, Propenylen oder Butenylen.

Besonders bevorzugt ist, dass X eine Alkylen- oder Alkylenoxy-Gruppe mit 2 bis 8 C-Atomen bedeutet. Hierbei sind geradkettige Gruppen besonders bevorzugt.

Im Sinne der vorliegenden Erfindung ist Z ein aromatisches oder heteroaromatisches Ringsystem mit 5 bis 60 Ringatomen, ausgewählt aus Indenofluoren, welches mit einem oder mehreren beliebigen Resten R substituiert sein kann. Z bildet den Grundkörper der Struktureinheit der allgemeinen Formel I und somit das sogenannte Polymergrundgerüst (Backbone).

In der Struktureinheit der allgemeinen Formel I sind Ar¹ und Ar² jeweils unabhängig voneinander eine Aryl- oder Heteroarylgruppe mit 5 bis 60 Ringatomen, welche mit einem oder mehreren beliebigen Resten R substituiert sein kann.

Eine Arylgruppe im Sinne der vorliegenden Erfindung enthält 5 bis 60 C-Atome, eine Heteroarylgruppe im Sinne der vorliegenden Erfindung enthält 2 bis 60 C-Atome und mindestens ein Heteroatom, mit der Maßgabe, dass die Summe aus C-Atomen und Heteroatomen mindestens 5 ergibt. Die Heteroatome sind vorzugsweise ausgewählt aus N, O und/oder S. Dabei wird unter einer Arylgruppe bzw. Heteroarylgruppe entweder ein einfacher aromatischer Cyclus, also Benzol, bzw. ein einfacher heteroaromatischer Cyclus, beispielsweise Pyridin, Pyrimidin, Thiophen, etc., oder eine kondensierte Aryl- oder Heteroarylgruppe, beispielsweise Naphthalin, Anthracen, Phenanthren, Chinolin, Isochinolin, Benzothiophen, Benzofuran und Indol etc., verstanden.

Besonders bevorzugt im Sinne der vorliegenden Erfindung sind die Reste Ar¹ und Ar² in der Struktureinheit der allgemeinen Formel I jeweils unabhängig voneinander abgeleitet von Benzol, Naphthalin, Pyridin, Anthracen, Phenanthren, Pyrimidin, Pyrazin, Pyridazin, Chinolin, Isochinolin, Furan, Thiophen, Pyrrol, Benzofuran, Benzothiophen und Indol, wobei insbesondere Benzol, Naphthalin, Pyridin, Anthracen, Phenanthren, Chinolin und Isochinolin bevorzugt ist.

In der Struktureinheit der allgemeinen Formel I ist R bei jedem Auftreten unabhängig voneinander ausgewählt aus F, Cl, Br, I, N(Ar)₂, N(R²)₂, CN, NO₂, Si(R²)₃, B(OR²)₂, C(=O)Ar, C(=O)R², P(=O)(Ar)₂, P(=O)(R²)₂, S(=O)Ar, S(=O)R², S(=O)₂Ar, S(=O)₂R², -CR²=CR²Ar, OSO₂R², einer geradkettigen Alkyl-, Alkoxy- oder Thioalkoxygruppe mit 1 bis 40 C-Atomen oder einer verzweigten oder cyclischen Alkyl-, Alkoxy- oder Thioalkoxygruppe mit 3 bis 40 C-Atomen, die jeweils mit einem oder mehreren Resten R² substituiert sein kann, wobei eine oder mehrere nicht benachbarte CH₂-Gruppen durch R²C=CR², C≡C, Si(R²)₂, Ge(R²)₂, Sn(R²)₂, C=O, C=S, C=Se, C=NR², P(=O)(R²), SO, SO₂, NR², O, S oder CONR² ersetzt sein können und wobei ein oder mehrere H-Atome durch F, Cl, Br, I, CN oder NO₂ ersetzt sein können, oder einem aromatischen oder heteroaromatischen Ringsystem mit 5 bis 60 Ringatomen, das jeweils durch einen oder mehrere Reste R² substituiert sein kann, oder einer Aryloxy- oder Heteroaryloxygruppe mit 5 bis 60 Ringatomen, die durch einen oder mehrere Reste R² substituiert sein kann, oder einer Kombination dieser Systeme, wobei zwei oder mehrere Substituenten R auch miteinander ein mono- oder polycyclisches, aliphatisches oder aromatisches Ringsystem bilden können, wobei R² jeweils unabhängig voneinander H oder ein aliphatischer oder aromatischer Kohlenwasserstoffrest mit 1 bis 20 C-Atomen ist.

Im Rahmen der vorliegenden Erfindung werden unter einer Alkylgruppe mit 1 bis 40 C-Atomen, in der auch einzelne H-Atome oder CH₂-Gruppen durch die oben genannten Gruppen oder Reste R substituiert sein können, bevorzugt die Reste Methyl, Ethyl, n-Propyl, i-Propyl, Cyclopropyl, n-Butyl, i-Butyl, s-Butyl, t-Butyl, Cyclobutyl, 2-Methylbutyl, n-Pentyl, s-Pentyl, Cyclopentyl, n-Hexyl, Cyclohexyl, n-Heptyl, Cycloheptyl, n-Octyl, Cyclooctyl, 2-Ethylhexyl, Trifluormethyl, Pentafluorethyl, 2,2,2-Trifluorethyl, Ethenyl, Propenyl, Butenyl, Pentenyl, Cyclopentenyl, Hexenyl, Cyclohexenyl, Heptenyl, Cycloheptenyl, Octenyl, Cyclooctenyl, Ethinyl, Propinyl, Butinyl, Pentinyl, Hexinyl oder Octinyl verstanden. Unter einer Alkoxygruppe mit 1 bis 40 C-Atomen werden vorzugsweise Methoxy, Trifluormethoxy, Ethoxy, n-Propoxy, i-Propoxy, n-Butoxy, i-Butoxy, s-Butoxy, t-Butoxy oder 2-Methylbutoxy verstanden.
Besonders bevorzugte Struktureinheiten der allgemeinen Formel I sind in der folgenden Tabelle aufgelistet:
In der Tabelle stellen die durchgezogenen Bindungsstriche (bei Z) die Verknüpfung zu den nächsten Struktureinheiten dar und die gestrichelten Bindungsstriche (bei Z, X, Ar¹, R¹, Ar² und Q) die Verknüpfung der einzelnen Elemente der Struktureinheit der allgemeinen Formel I dar, wobei die einzelnen Elemente jeweils durch eine Einfachbindung miteinander verknüpft sind. Die Einträge 1,7 und 8 der Tabelle stellen Referenzeinträge dar.

| | Z | X | Ar¹ | R¹ | Ar² | Q | n |
|---|---|---|---|---|---|---|---|
| 1 | 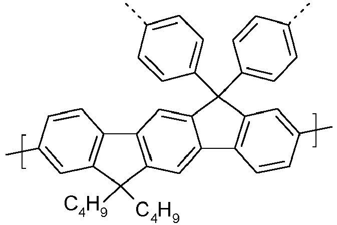 | ---O--- | 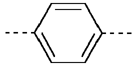 | 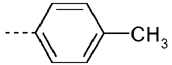 | 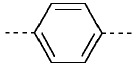 | 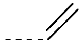 | 2 |
| 2 | 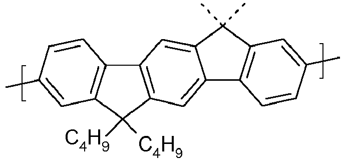 | direkte Bindung | 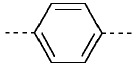 | 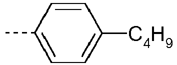 | 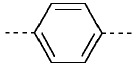 | 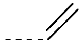 | 2 |
| 3 | 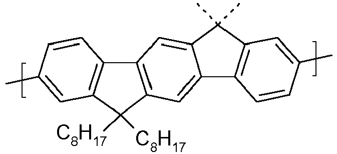 | direkte Bindung | 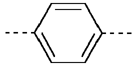 | 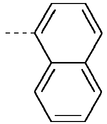 | 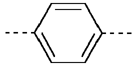 | 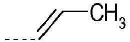 | 2 |
| 4 | 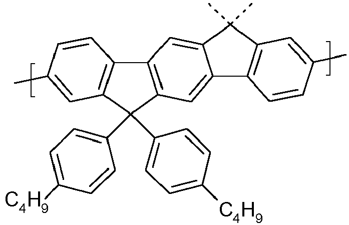 | 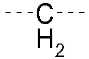 | 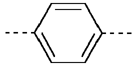 | 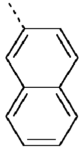 | 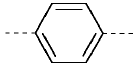 | 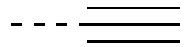 | 2 |
| 5 | 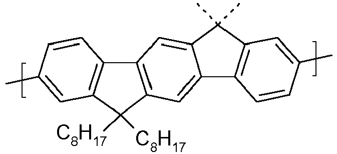 | ----CH₂-CH₂-O ---- | 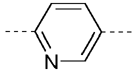 | 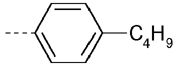 | 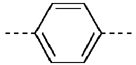 | 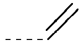 | 2 |
| 6 | 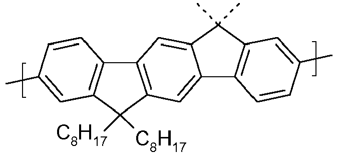 | direkte Bindung | 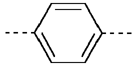 | 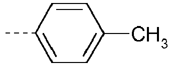 | 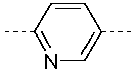 | 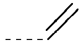 | 2 |
| 7 | 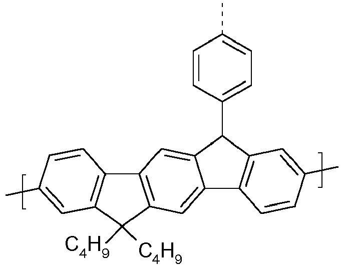 | 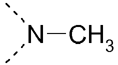 | 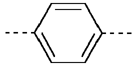 | 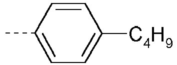 | 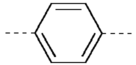 | 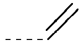 | 1 |
| 8 | 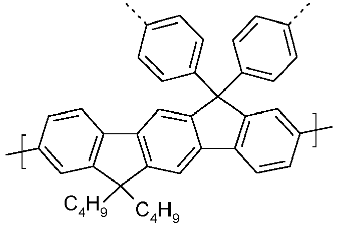 | ---O--- | 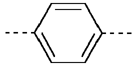 | 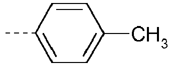 | 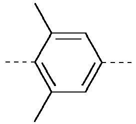 | 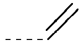 | 2 |
| 9 | 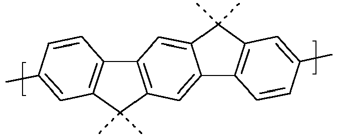 | direkte Bindung | 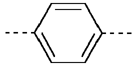 | 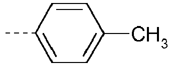 | 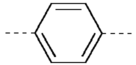 | 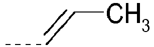 | 4 |
| 10 | 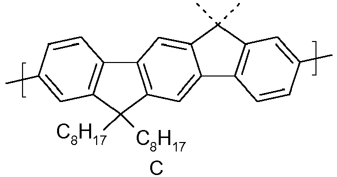 | direkte Bindung | 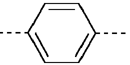 | 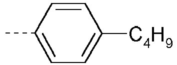 | 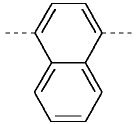 | 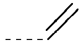 | 2 |

Unter einem Polymer im Sinne der vorliegenden Erfindung soll auch ein Oligomer und ein Dendrimer verstanden werden.

Als Oligomer wird im Sinne der vorliegenden Erfindung eine Verbindung bezeichnet, welche drei bis neun Wiederholungseinheiten aufweist. Als Polymer im Sinne der vorliegenden Erfindung wird eine Verbindung verstanden, welche zehn oder mehr Wiederholungseinheiten aufweist. Der Verzweigungs-Faktor der Polymeren liegt dabei zwischen 0 (lineares Polymer, ohne Verzweigungsstellen) und 1 (vollständig verzweigtes Dendrimer).

Die Polymere, Oligomere oder Dendrimere können konjugiert, teilkonjugiert oder nicht-konjugiert sein. Die Polymere oder Oligomere können linear, verzweigt oder dendritisch sein. In den linear verknüpften Strukturen können die Einheiten der allgemeinen Formel I sowohl direkt miteinander verknüpft sein oder sie können über eine bivalente Gruppe, beispielsweise über eine substituierte oder unsubstituierte Alkylengruppe, über ein Heteroatom oder über eine bivalente aromatische oder heteroaromatische Gruppe, miteinander verknüpft sein. In verzweigten Strukturen können beispielsweise drei oder mehrere Einheiten der allgemeinen Formel I über eine trivalente oder höhervalente Gruppe, beispielsweise über eine trivalente oder höhervalente aromatische oder heteroaromatische Gruppe, zu einem verzweigten Polymer oder Oligomer verknüpft sein.

Der Anteil der Struktureinheit der allgemeinen Formel I im Polymer liegt im Bereich von 0,1 bis 50 mol% und vorzugsweise im Bereich von 0,5 bis 30 mol%.

Die erfindungsgemäßen Polymere können neben einer oder mehrerer Struktureinheiten der allgemeinen Formel I noch weitere Struktureinheiten enthalten. Dies sind u. a. solche, wie sie in der WO 02/077060 A1 und in der WO 2005/014689 A2 offenbart und umfangreich aufgelistet sind. Diese werden via Zitat als Bestandteil der vorliegenden Erfindung betrachtet. Die weiteren Struktureinheiten können beispielsweise aus den folgenden Klassen stammen:

| | |
|---|---|
| Gruppe 1: | Einheiten, welche die Lochinjektions- und/oder Lochtransporteigenschaften der Polymere erhöhen; |
| Gruppe 2: | Einheiten, welche die Elektroneninjektions- und/oder Elektronentransporteigenschaften der Polymere erhöhen; |
| Gruppe 3: | Einheiten, die Kombinationen von Einzeleinheiten der Gruppe 1 und Gruppe 2 aufweisen; |
| Gruppe 4: | Einheiten, welche die Emissionscharakteristik insoweit verändern, dass Elektrophosphoreszenz statt Elektrofluoreszenz erhalten werden kann; |
| Gruppe 5: | Einheiten, welche den Übergang vom so genannten Singulett- zum Triplettzustand verbessern; |
| Gruppe 6: | Einheiten, welche die Emissionsfarbe der resultierenden Polymere beeinflussen; |
| Gruppe 7: | Einheiten, welche typischerweise als Backbone verwendet werden; |
| Gruppe 8: | Einheiten, welche die Filmmorphologie und/oder die rheologischen Eigenschaften der resultierenden Polymere beeinflussen. |

Bevorzugte erfindungsgemäße Polymere sind solche, bei denen mindestens eine Struktureinheit Ladungstransporteigenschaften aufweist, d. h. die Einheiten aus der Gruppe 1 und/oder 2 enthalten.

Struktureinheiten aus der Gruppe 1, die Lochinjektions- und/oder Lochtransporteigenschaften aufweisen, sind beispielsweise Triarylamin-, Benzidin-, Tetraaryl-para-phenylendiamin-, Triarylphosphin-, Phenothiazin-, Phenoxazin-, Dihydrophenazin-, Thianthren-, Dibenzopara-dioxin-, Phenoxathiin-, Carbazol-, Azulen-, Thiophen-, Pyrrol- und Furanderivate und weitere O-, S- oder N-haltige Heterocyclen mit hoch liegendem HOMO (HOMO = höchstes besetztes Molekülorbital). Vorzugsweise führen diese Arylamine und Heterocyclen zu einem HOMO im Polymer von mehr als -5,8 eV (gegen Vakuumlevel), besonders bevorzugt von mehr als -5,5 eV.

Struktureinheiten aus der Gruppe 2, die Elektroneninjektions- und/oder Elektronentransporteigenschaften aufweisen, sind beispielsweise Pyridin-, Pyrimidin-, Pyridazin-, Pyrazin-, Oxadiazol-, Chinolin-, Chinoxalin-, Anthracen-, Benzanthracen-, Pyren-, Perylen-, Benzimidazol-, Triazin-, Keton-, Phosphinoxid- und Phenazinderivate, aber auch Triarylborane und weitere O-, S- oder N-haltige Heterocyclen mit niedrig liegendem LUMO (LUMO = niedrigstes unbesetztes Molekülorbital). Vorzugsweise führen diese Einheiten im Polymer zu einem LUMO von weniger als -2,5 eV (gegen Vakuumlevel), besonders bevorzugt von weniger als -2,7 eV.

Es kann bevorzugt sein, wenn in den erfindungsgemäßen Polymeren Einheiten aus der Gruppe 3 enthalten sind, in denen Strukturen, welche die Lochmobilität und welche die Elektronenmobilität erhöhen (also Einheiten aus Gruppe 1 und 2), direkt aneinander gebunden sind oder Strukturen enthalten sind, die sowohl die Lochmobilität als auch die Elektronenmobilität erhöhen. Einige dieser Einheiten können als Emitter dienen und verschieben die Emissionsfarbe ins Grüne, Gelbe oder Rote. Ihre Verwendung eignet sich also beispielsweise für die Erzeugung anderer Emissionsfarben aus ursprünglich blau emittierenden Polymeren.

Struktureinheiten aus der Gruppe 4 sind solche, welche auch bei Raumtemperatur mit hoher Effizienz aus dem Triplettzustand Licht emittieren können, also Elektrophosphoreszenz statt Elektrofluoreszenz zeigen, was häufig eine Steigerung der Energieeffizienz bewirkt. Hierfür eignen sich zunächst Verbindungen, welche Schweratome mit einer Ordnungszahl von mehr als 36 enthalten. Bevorzugt sind Verbindungen, welche d- oder f-Übergangsmetalle enthalten, die die o. g. Bedingung erfüllen. Besonders bevorzugt sind hier entsprechende Struktureinheiten, welche Elemente der Gruppe 8 bis 10 (Ru, Os, Rh, Ir, Pd, Pt) enthalten. Als Struktureinheiten für die erfindungsgemäßen Polymeren kommen hier z.B. verschiedene Komplexe in Frage, wie sie z.B. in der WO 02/068435 A1, der WO 02/081488 A1, der EP 1239526 A2 und der WO 04/026886 A2 beschrieben werden. Entsprechende Monomere werden in der WO 02/068435 A1 und in der WO 05/042548 A1 beschrieben.

Struktureinheiten der Gruppe 5 sind solche, welche den Übergang vom Singulett- zum Triplettzustand verbessern und welche, unterstützend zu den Strukturelementen der Gruppe 4 eingesetzt, die Phosphoreszenzeigenschaften dieser Strukturelemente verbessern. Hierfür kommen insbesondere Carbazol- und überbrückte Carbazoldimereinheiten in Frage, wie sie z.B. in der WO 04/070772 A2 und der WO 04/113468 A1 beschrieben werden. Weiterhin kommen hierfür Ketone, Phosphinoxide, Sulfoxide, Sulfone, Silan-Derivate und ähnliche Verbindungen in Frage, wie sie z.B. in der WO 05/040302 A1 beschrieben werden.

Struktureinheiten der Gruppe 6 sind neben den oben genannten solche, die mindestens noch eine weitere aromatische oder eine andere konjugierte Struktur aufweisen, welche nicht unter die o. g. Gruppen fallen, d. h. die die Ladungsträgermobilitäten nur wenig beeinflussen, die keine metallorganischen Komplexe sind oder die keinen Einfluss auf den Singulett-Triplett-Übergang haben. Derartige Strukturelemente können die Emissionsfarbe der resultierenden Polymere beeinflussen. Je nach Einheit können sie daher auch als Emitter eingesetzt werden. Bevorzugt sind dabei aromatische Strukturen mit 6 bis 40 C-Atomen oder auch Tolan-, Stilben- oder Bisstyrylarylenderivate, die jeweils mit einem oder mehreren Resten R substituiert sein können. Besonders bevorzugt ist dabei der Einbau von 1,4-Phenylen-, 1,4-Naphthylen-, 1,4- oder 9,10-Anthrylen-, 1,6-, 2,7- oder 4,9-Pyrenylen-, 3,9- oder 3,10-Perylenylen-, 4,4'-Biphenylylen-, 4,4"-Terphenylylen, 4,4'-Bi-1,1'-naphthylylen-, 4,4'-Tolanylen-, 4,4'-Stilbenylen-, 4,4"-Bisstyrylarylen-, Benzothiadiazol- und entsprechenden Sauerstoffderivaten, Chinoxalin-, Phenothiazin-, Phenoxazin-, Dihydrophenazin-, Bis(thiophenyl)arylen-, Oligo(thiophen-ylen)-, Phenazin-, Rubren-, Pentacen- oder Perylenderivaten, die vorzugsweise substituiert sind, oder vorzugsweise konjugierte Push-Pull-Systeme (Systeme, die mit Donor- und Akzeptorsubstituenten substituiert sind) oder Systeme wie Squarine oder Chinacridone, die vorzugsweise substituiert sind.

Struktureinheiten der Gruppe 7 sind Einheiten, die aromatische Strukturen mit 6 bis 40 C-Atomen beinhalten, welche typischerweise als Polymergrundgerüst (Backbone) verwendet werden. Dies sind beispielsweise 4,5-Dihydropyrenderivate, 4,5,9,10-Tetrahydropyrenderivate, Fluorenderivate, 9,9'-Spirobifluorenderivate, Phenanthrenderivate, 9,10-Dihydrophenanthrenderivate, 5,7-Dihydrodibenzooxepinderivate und cis- und trans-Indenofluorenderivate.

Struktureinheiten der Gruppe 8 sind solche, die die Filmmorphologie und/oder die rheologischen Eigenschaften der Polymere beeinflussen, wie z.B. Siloxane, lange Alkylketten oder fluorierte Gruppen, aber auch besonders steife oder flexible Einheiten, wie z.B. flüssigkristallbildende Einheiten oder vernetzbare Gruppen.

Bevorzugt sind erfindungsgemäße Polymere, die gleichzeitig neben den Struktureinheiten der allgemeinen Formel I zusätzlich noch ein oder mehrere Einheiten ausgewählt aus den Gruppen 1 bis 8 enthalten, die von den erfindungsgemäßen Struktureinheiten verschieden sind. Es kann ebenfalls bevorzugt sein, wenn gleichzeitig mehr als eine Struktureinheit aus einer Gruppe vorliegt.

Bevorzugt sind dabei erfindungsgemäße Polymere, die neben mindestens einer Struktureinheit der allgemeinen Formel I noch Einheiten aus der Gruppe 7 enthalten, besonders bevorzugt mindestens 50 mol% dieser Einheiten, bezogen auf die Gesamtzahl der Struktureinheiten im Polymer.

Ebenfalls bevorzugt ist es, wenn die erfindungsgemäßen Polymere Einheiten enthalten, die den Ladungstransport oder die Ladungsinjektion verbessern, also Einheiten aus der Gruppe 1 und/oder 2; besonders bevorzugt ist ein Anteil von 0,5 bis 30 mol% dieser Einheiten; ganz besonders bevorzugt ist ein Anteil von 1 bis 10 mol% dieser Einheiten.

Besonders bevorzugt ist es weiterhin, wenn die erfindungsgemäßen Polymere Struktureinheiten aus der Gruppe 7 und Einheiten aus der Gruppe 1 und/oder 2 enthalten, insbesondere mindestens 50 mol% Einheiten aus der Gruppe 7 und 0,5 bis 30 mol% Einheiten aus der Gruppe 1 und/oder 2.

Die erfindungsgemäßen Polymere sind Copolymere. Die erfindungsgemäßen Polymere können dabei linear oder verzweigt sein. Erfindungsgemäße Copolymere können dabei neben einer oder mehreren Struktureinheiten der allgemeinen Formel I oder deren bevorzugten Unterformeln, potentiell eine oder mehrere weitere Strukturen aus den oben aufgeführten Gruppen 1 bis 8 besitzen.

Das vernetzbare Polymer, Oligomer oder Dendrimer kann durch Beschichtung aus Lösung auf ein entsprechendes Trägersubstrat (Glas, Polymer etc.) bzw. eine bereits davor abgeschiedene Schicht aufgebracht werden und entweder vor oder nach Entfernung des Lösungsmittels vernetzt werden. Die vernetzbare Gruppe Q am Aromaten, insbesondere die Vinylgruppe, weist dabei eine ausreichende Reaktivität auf, so dass weniger energiereiche UV-Strahlung oder eine geringere thermische Energiemenge zur Vernetzung notwendig ist. Die Vernetzungsreaktion ist somit für die Polymerschicht schonender als die im Stand der Technik bekannten Vernetzungsverfahren mit energiereicher Strahlung oder thermischer Energie (meist über 200°C). Das erfindungsgemäße Polymer weist jedoch auch eine hohe Stabilität auf, so dass es bei Vernetzungsmethoden gemäß dem Stand der Technik nicht oder nur in geringerem Umfang zur Bildung von unerwünschten Nebenprodukten kommt, welche die elektrooptischen Eigenschaften negativ beeinflussen könnten.

Zur Herstellung der erfindungsgemäßen Polymere werden Monomere eingesetzt.

Dazu wird eine Verbindung der allgemeinen Formel II bereitgestellt, 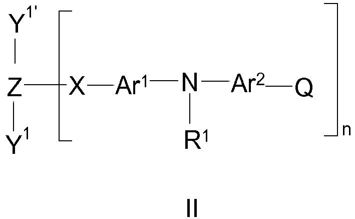 wobei
Z, X, Ar¹, Ar², Q, R, R¹ und n die in Bezug auf Formel (I) angegebenen Bedeutungen annehmen können, und
Y¹, Y^{1'} jeweils Abgangsgruppen sind, die gleich oder verschieden sein können.

Bevorzugt sind die Reste Ar¹ und Ar² in der allgemeinen Formel II jeweils unabhängig voneinander abgeleitet von Benzol, Naphthalin, Pyridin, Anthracen, Phenanthren, Pyrimidin, Pyrazin, Pyridazin, Chinolin, Isochinolin, Furan, Thiophen, Pyrrol, Benzofuran, Benzothiophen und Indol, wobei insbesondere Benzol, Naphthalin, Pyridin, Anthracen, Phenanthren, Chinolin und Isochinolin bevorzugt ist.

In der Verbindung der allgemeinen Formel II ist R bei jedem Auftreten unabhängig voneinander ausgewählt aus F, Cl, Br, I, N(Ar)₂, N(R²)₂, CN, NO₂, Si(R²)₃, B(OR²)₂, C(=O)Ar, C(=O)R², P(=O)(Ar)₂, P(=O)(R²)₂, S(=O)Ar, S(=O)Ar², S(O)₂Ar, S(=O)R², S(=O)₂Ar, S(=O)₂R², -CR²=CR²Ar, OSO₂R², einer geradkettigen Alkyl-, Alkoxy- oder Thioalkoxygruppe mit 1 bis 40 C-Atomen oder einer verzweigten oder cyclischen Alkyl-, Alkoxy- oder Thioalkoxygruppe mit 3 bis 40 C-Atomen, die jeweils mit einem oder mehreren Resten R² substituiert sein kann, wobei eine oder mehrere nicht benachbarte CH₂-Gruppen durch R²C=CR², C≡C, Si(R²)₂, Ge(R²)₂, Sn(R²)₂, C=O, C=S, C=Se, C=NR², P(=O)(R²), SO, SO₂, NR², O, S oder CONR² ersetzt sein können und wobei ein oder mehrere H-Atome durch F, Cl, Br, I, CN oder NO₂ ersetzt sein können, oder einem aromatischen oder heteroaromatischen Ringsystem mit 5 bis 60 Ringatomen, das jeweils durch einen oder mehrere Reste R² substituiert sein kann, oder einer Aryloxy- oder Heteroaryloxygruppe mit 5 bis 60 Ringatomen, die durch einen oder mehrere Reste R² substituiert sein kann, oder einer Kombination dieser Systeme, wobei zwei oder mehrere Substituenten R auch miteinander ein mono- oder polycyclisches, aliphatisches oder aromatisches Ringsystem bilden können, wobei R² jeweils unabhängig voneinander H oder ein aliphatischer oder aromatischer Kohlenwasserstoffrest mit 1 bis 20 C-Atomen ist.

Die Abgangsgruppen Y¹ und Y^{1'} sind jeweils unabhängig voneinander, gleich oder verschieden, Abgangsgruppen, die vorzugsweise einer metallkatalysierten Kreuzkupplungsreaktion zugänglich sind. Die mit den Abgangsgruppen funktionalisierten Verbindungen stellen die Basis für eine Polymerisation dar. So können Bromderivate mit Arylboronsäuren oder Arylboronsäurederivaten gemäß Suzuki-Kupplung oder mit Organozinnverbindungen gemäß Stille zu den entsprechenden Polymeren, Oligomeren oder Dendrimeren umgesetzt werden.

Diese Verfahren sind im Stand der Technik bekannt. So handelt es sich bei der Suzuki-Kupplung beispielsweise um eine Kreuzkupplungsreaktion, beispielsweise zur Bildung von Diphenylderivaten, wobei vorzugsweise Arylboronsäuren mit Halogenaromaten unter katalytischer Verwendung von vorzugsweise Palladiumphosphankomplexen umgesetzt werden. Die Reaktivität der Aromaten steigt dabei von Brom über Trifluormethansulfonsäureesther bis zum Iod, wobei mittlerweile selbst schwach reaktive Chloraromaten mit Palladium-Phosphan Katalysatoren umgesetzt werden können. Analog verläuft die Kreuzkupplungsreaktion gemäß Stille, wobei anstatt von Bororganylen auf Organozinnverbindungen zurückgegriffen wird, die jedoch aufgrund ihrer hohen Toxizität nicht bevorzugt sind.

Vorzugsweise sind Y¹ und Y^{1'} jeweils unabhängig voneinander ausgewählt aus Halogenid, Borsäure, Borsäureester, Boran, Mesitylat und Triflat. Das Halogenid ist dabei bevorzugt F, Cl, Br, I, besonders bevorzugt Br.

Bei einem Verfahren zur Herstellung einer Verbindung der allgemeinen Formel II 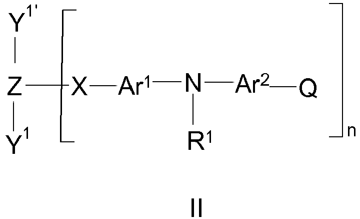 wie oben definiert, werden in einen Grundkörper Z reaktive Abgangsgruppen Y¹, Y^{1'} und ein oder mehrere Reste 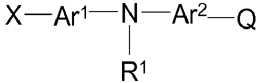 eingebracht, wobei die Symbole und Indices die oben angegebenen Bedeutungen haben. Q ist eine Vinyl- oder Alkenylgruppe.

Es ist dabei vom Reaktionsweg unabhängig, ob beispielsweise zuerst Y¹ und Y^{1'} und dann der die vernetzbare Gruppe tragende Rest in den Grundkörper Z eingebracht wird oder umgekehrt. Zur Einbringung der Reste Y¹ bzw. Y^{1'} gibt es mehrere Standardverfahren der organischen Chemie. Für den Fall, dass Y¹ und/oder Y^{1'} Brom bedeutet, kann dies beispielsweise durch eine Bromierung mit Br₂ oder beispielsweise NBS (N-Bromsuccinimid) erfolgen. Auf diese Art lässt sich beispielsweise auch selektiv eine Mono- oder Dibromierung steuern. Auch die Einbringung des Restes, d. h. die Kopplung von X bzw. Ar¹ an Z, kann nach Standardmethoden der organischen Chemie erfolgen. Alternativ ist auch vorstellbar, dass beispielsweise zuerst der mit Y¹ und Y^{1'} substituierte Grundkörper Z polymerisiert wird und anschließend der Rest entweder über X oder direkt über Ar¹ an die Polymerhauptkette gepfropft wird.

Die Abgangsgruppen Y¹ und Y^{1'} können für eine Polymerisation der Verbindungen der allgemeinen Formel II zum Polymer, Oligomer oder Dendrimer genutzt werden. Hierzu sind insbesondere solche Verbindungen bevorzugt, welche mit reaktiven Abgangsgruppen, wie Brom, Iod, Boronsäure oder Boronsäureester substituiert sind. Diese können auch als Comonomere zur Erzeugung entsprechender konjugierter, teilkonjugierter oder nicht-konjugierter Polymere, Oligomere oder auch als Kern von Dendrimeren Verwendung finden. Die Polymerisation erfolgt dabei vorzugsweise über die Halogenfunktionalität bzw. die Boronsäurefunktionalität.

Zur Herstellung der Polymere, Oligomere oder Dendrimere werden die funktionalisierten Verbindungen der allgemeinen Formel II mit weiteren Monomeren copolymerisiert.

Geeignete Polymerisationsreaktionen sind dem Fachmann bekannt und in der Literatur beschrieben. Besonders geeignete und bevorzugte Polymerisations- und Kupplungsreaktionen, die alle zu C-C-Verknüpfungen führen, sind solche gemäß SUZUKI, YAMAMOTO, STILLE, HECK, NEGISHI, SONOGASHIRA oder HIYAMA. Die C-C-Verknüpfungen werden besonders bevorzugt über eine SUZUKI-Kupplung, YAMAMOTO-Kupplung oder STILLE-Kupplung durchgeführt.

Wie die Polymerisation nach diesen Methoden durchgeführt werden kann und wie die Polymere dann vom Reaktionsmedium abgetrennt und gereinigt werden können, ist dem Fachmann bekannt und in der Literatur, beispielsweise in der WO 03/048225 und der WO 04/037887, im Detail beschrieben.

Da Copolymere hergestellt werden, ist es besonders bevorzugt, wenn die Verbindung gemäß der allgemeinen Formel II im Bereich von 0,5 bis 30 Mol-% vorhanden ist. Geeignete und bevorzugte Comonomere sind ausgewählt aus Fluorenen (z. B. gemäß EP 842208 oder WO 00/22026), Spirobifluorenen (z. B. gemäß EP 707020, EP 894107 oder WO 06/061181), Para-phenylenen (z. B. gemäß WO 92/18552), Carbazolen (z. B. gemäß WO 04/070772 oder WO 04/113468), Thiophenen (z. B. gemäß EP 1028136), Dihydrophenanthrenen (z. B. gemäß WO 05/014689), cis- und trans-Indenofluorenen (z. B. gemäß WO 04/041901 oder WO 04/113412), Ketonen (z. B. gemäß WO 05/040302), Phenanthrenen (z. B. gemäß WO 05/104264 oder DE 102005037734), Benzanthrene, Benzanthracene oder auch mehreren dieser Einheiten. Diese Polymere enthalten üblicherweise noch weitere Einheiten, beispielsweise emittierende (fluoreszierende oder phosphoreszierende) Einheiten oder phosphoreszierende Metallkomplexe (z. B. gemäß WO 06/003000), und/oder Ladungstransporteinheiten, insbesondere solche basierend auf Triarylaminen. Besonders bevorzugt sind Monomere, welche Struktureinheiten aufweisen, die aus den oben genannten Gruppen 1 bis 8 ausgewählt sind.

Polymere, Oligomere oder Dendrimere, welche die erfindungsgemäßen Struktureinheiten enthalten, finden beispielsweise zur Herstellung von OLEDs bzw. PLEDs Verwendung, vorzugsweise als Emitterschicht, Elektronentransportschicht, Elektroneninjektionsschicht, Lochinjektionsschicht und/oder Lochtransportschicht.

Die Erzeugung der Polymerschicht kann beispielsweise durch Beschichtung aus Lösung, vorzugsweise Spin-Coating erfolgen. Nach dem Aufbringen der Polymerschicht und Entfernen des Lösungsmittels kann das Polymer vernetzt werden. Die Vernetzung erfolgt vorzugsweise strahlungsinduziert (z.B. mit UV-Licht, sichtbarem Licht, Mikrowellen, Elektronenstrahlen) oder thermisch, vorzugsweise bei Temperaturen von weniger als 200°C.

Gegenstand der Erfindung ist auch die Verwendung der Polymere, Oligomere oder Dendrimere in einer organischen, elektronischen Vorrichtung.

Die organische, elektronische Vorrichtung ist vorzugsweise eine organische elektrolumineszierende Vorrichtung (OLED), eine polymere elektrolumineszierende Vorrichtung (PLED), eine organische integrierte Schaltung (O-IC), ein organischer Feld-Effekt-Transistor (O-FT), ein organischer Dünnfilmtransistor (O-TFT), ein organischer, lichtemittierender Transistor (O-LET), eine organische Solarzelle (O-SC), ein organischer, optischer Detektor, ein organischer Fotorezeptor, ein organisches Feld-Quench-Device (O-FQD), eine lichtemittierende elektrochemische Zelle (LEC) oder eine organische Laserdiode (O-Laser).

Im Sinne der vorliegenden Erfindung ist es bevorzugt, dass das erfindungsgemäße Polymer, Oligomer oder Dendrimer als Schicht (oder in einer Schicht) in der elektronischen Vorrichtung vorliegt.

Weiterhin ist bevorzugt, dass das Polymer, Oligomer oder Dendrimer vernetzt ist.

Die Copolymere können ferner linear oder verzweigt sein. Die erfindungsgemäßen Copolymere können statistische, alternierende oder blockartige Strukturen aufweisen oder auch mehrere dieser Strukturen abwechselnd besitzen. Wie Copolymere mit blockartigen Strukturen erhalten werden können und welche weiteren Strukturelemente dafür besonders bevorzugt sind, ist beispielsweise ausführlich in der WO 05/014688 beschrieben.

In einer weiteren Ausführungsform der vorliegenden Erfindung umfasst die Vorrichtung mehrere Schichten. Das erfindungsgemäße Polymer, Oligomer oder Dendrimer kann dabei in Form einer Lochtransport-, Lochinjektions-, Emitter-, Elektronentransport-, Elektroneninjektions-, Ladungsblockier- und/oder Ladungserzeugungsschicht vorliegen.

Die Vorrichtung kann ferner Schichten enthalten, welche aus kleinen Molekülen aufgebaut sind (SMOLED). Diese können durch Verdampfen von kleinen Molekülen im Hochvakuum erzeugt werden.

Es kann außerdem bevorzugt sein, das Polymer nicht als Reinsubstanz, sondern als Mischung (Blend) zusammen mit weiteren beliebigen polymeren, oligomeren, dendritischen oder niedermolekularen Substanzen zu verwenden. Diese können beispielsweise die elektronischen Eigenschaften verbessern oder selber emittieren. Solche Blends sind daher auch Gegenstand der vorliegenden Erfindung.

In einer bevorzugten Ausführungsform der vorliegenden Erfindung werden die erfindungsgemäßen Polymere, Oligomere oder Dendrimere als emittierende Verbindungen in einer emittierenden Schicht eingesetzt.

Dabei kann die organische Elektrolumineszenzvorrichtung eine emittierende Schicht enthalten oder sie kann mehrere emittierende Schichten enthalten, wobei mindestens eine emittierende Schicht mindestens ein erfindungsgemäßes Polymer, Oligomer oder Dendrimer, wie oben definiert, enthält oder daraus besteht. Wenn mehrere Emissionsschichten vorhanden sind, weisen diese vorzugsweise insgesamt mehrere Emissionsmaxima zwischen 380 nm und 750 nm auf, so dass insgesamt weiße Emission resultiert, d. h. in den emittierenden Schichten werden verschiedene emittierende Verbindungen verwendet, die fluoreszieren oder phosphoreszieren können. Insbesondere bevorzugt sind Dreischichtsysteme, wobei die drei Schichten blaue, grüne und orange oder rote Emission zeigen (für den prinzipiellen Aufbau siehe z. B. WO 05/011013). Weiß emittierende Vorrichtungen eignen sich z.B. als Beleuchtung oder Hintergrundbeleuchtung von Displays (LCD).

Außer diesen Schichten kann die organische Elektrolumineszenzvorrichtung noch weitere Schichten enthalten, beispielsweise jeweils eine oder mehrere Lochinjektionsschichten, Lochtransportschichten, Lochblockierschichten, Elektronentransportschichten, Elektroneninjektionsschichten, Excitonenblockierschichten und/oder Ladungserzeugungsschichten (Charge-Generation Layers, IDMC 2003, Taiwan; Session 21 OLED (5), T. Matsumoto, T. Nakada, J. Endo, K. Mori, N. Kawamura, A. Yokoi, J. Kido, Multiphoton Organic EL Device Having Charge Generation Layer). Ebenso können zwischen zwei emittierende Schichten Interlayer eingebracht sein, welche beispielsweise eine Excitonen-blockierende Funktion aufweisen. Es sei aber darauf hingewiesen, dass nicht notwendigerweise jede dieser Schichten vorhanden sein muss. Diese Schichten können ebenfalls die erfindungsgemäßen Polymere, Oligomere oder Dendrimere, wie oben definiert, enthalten. Möglich ist auch, dass mehrere OLEDs übereinander angeordnet werden, wodurch eine weitere Effizienzsteigerung hinsichtlich der Lichtausbeute erreicht werden kann. Zur Verbesserung der Lichtauskopplung kann die letzte organische Schicht auf der Lichtaustrittseite bei OLEDs beispielsweise auch als Nanoschaum ausgeführt sein, wodurch der Anteil der Totalreflexion verringert wird.

Weiterhin bevorzugt ist eine organische Elektrolumineszenzvorrichtung, wobei eine oder mehrere Schichten mit einem Sublimationsverfahren beschichtet werden. Dabei werden die Materialien in Vakuum-Sublimationsanlagen bei einem Druck kleiner 10⁻⁵ mbar, vorzugsweise kleiner 10⁻⁶ mbar, besonders bevorzugt kleiner 10⁻⁷ mbar aufgedampft.

Bevorzugt ist ebenfalls eine organische Elektrolumineszenzvorrichtung, die dadurch gekennzeichnet ist, dass eine oder mehrere Schichten mit dem OVPD (Organic Vapour Phase Deposition) Verfahren oder mit Hilfe einer Trägergassublimation beschichtet werden. Dabei werden die Materialien bei einem Druck zwischen 10⁻⁵ mbar und 1 bar aufgebracht.

Weiterhin bevorzugt ist eine organische Elektrolumineszenzvorrichtung, die dadurch gekennzeichnet ist, dass eine oder mehrere Schichten aus Lösung, wie z. B. durch Spincoating, oder mit einem beliebigen Druckverfahren, wie z. B. Siebdruck, Flexodruck oder Offsetdruck, besonders bevorzugt aber LITI (Light Induced Thermal Imaging, Thermotransferdruck) oder Ink-Jet Druck (Tintenstrahldruck), hergestellt werden. Hierfür sind lösliche Verbindungen nötig, welche gegebenenfalls durch geeignete Substitution erhalten werden.

Entsprechend ist ein weiterer Gegenstand der vorliegenden Erfindung eine Formulierung, enthaltend ein Polymer, Oligomer oder Dendrimer, wie oben definiert, das Struktureinheiten der allgemeinen Formel I aufweist, in einem oder mehreren Lösungsmitteln. Wie solche Formulierungen hergestellt werden können, ist dem Fachmann bekannt und z.B. in der WO 02/072714, der WO 03/019694 und der darin zitierten Literatur beschrieben.

Geeignete und bevorzugte Lösungsmittel sind beispielsweise Toluol, Anisol, Xylole, Methylbenzoat, Dimethylanisole, Mesitylene, Tetralin, Veratrol, Tetrahydrofuran und Chlorbenzol sowie Gemische derselben.

Die Vorrichtung enthält gewöhnlich eine Kathode und eine Anode (Elektroden). Die Elektroden (Kathode, Anode) werden im Sinne der vorliegenden Erfindung so gewählt, dass ihr Potential möglichst gut mit dem Potential der angrenzenden, organischen Schicht übereinstimmt, um eine möglichst effiziente Elektronen- bzw. Lochinjektion zu gewährleisten.

Als Kathode sind Metallkomplexe, Metalle mit geringer Austrittsarbeit, Metalllegierungen oder mehrlagige Strukturen aus verschiedenen Metallen bevorzugt, wie beispielsweise Erdalkalimetalle, Alkalimetalle, Hauptgruppenmetalle oder Lathanoide (z. B. Ca, Ba, Mg, Al, In, Mg, Yb, Sm, etc.). Bei mehrlagigen Strukturen können auch zusätzlich zu den genannten Metallen weitere Metalle verwendet werden, die eine relativ hohe Austrittsarbeit aufweisen, wie z. B. Ag, wobei dann in der Regel Kombinationen der Metalle, wie beispielsweise Ca/Ag oder Ba/Ag verwendet werden. Es kann auch bevorzugt sein, zwischen einer metallischen Kathode und dem organischen Halbleiter eine dünne Zwischenschicht eines Materials mit einer hohen Dielektrizitätskonstante einzubringen. Hierfür kommen beispielsweise Alkalimetall- oder Erdalkalimetallfluoride, aber auch die entsprechenden Oxide in Frage (z. B. LiF, Li₂O, BaF2, MgO, NaF, etc.). Die Schichtdicke dieser Schicht liegt vorzugsweise zwischen 1 und 10 nm, besonders bevorzugt zwischen 2 und 8 nm.

Als Anode sind Materialien mit hoher Austrittsarbeit bevorzugt. Vorzugsweise weist die Anode ein Potential größer 4,5 eV vs. Vakuum auf. Hierfür sind einerseits Metalle mit hohem Redoxpotential geeignet, wie beispielsweise Ag, Pt oder Au. Es können andererseits auch Metall/Metalloxid-Elektroden (z. B. Al/Ni/NiOₓ, Al/PtOₓ) bevorzugt sein. Für einige Anwendungen muss mindestens eine der Elektroden transparent sein, um entweder die Bestrahlung des organischen Materials (O-SC) oder die Auskopplung von Licht (OLED/PLED, O-LASER) zu ermöglichen. Ein bevorzugter Aufbau verwendet eine transparente Anode. Bevorzugte Anodenmaterialien sind hier leitfähige, gemischte Metalloxide. Besonders bevorzugt sind Indium-Zinn-Oxid (ITO) oder Indium-Zink Oxid (IZO). Bevorzugt sind weiterhin leitfähige, dotierte organische Materialien, insbesondere leitfähige dotierte Polymere, wie z.B Poly(ethylendioxythiophen) (PEDOT) und Polyanilin (PANI).

Die Vorrichtung wird in an sich bekannter Weise je nach Anwendung entsprechend strukturiert, kontaktiert und schließlich hermetisch versiegelt, da sich die Lebensdauer derartiger Vorrichtungen bei Anwesenheit von Wasser und/oder Luft drastisch verkürzt.

Die Erfindung wird im folgenden anhand von Ausführungsbeispielen näher erläutert, ohne dadurch jedoch beschränkt zu werden.

### Beispiele:

### A) Herstellung der Monomeren

### Beispiel 1:

### Herstellung von Monomer 1:

Die Herstellung von Monomer 1 folgt dem unten abgebildeten Schema 1. Die Umsetzungen in den einzelnen Stufen erfolgen dabei nach dem Fachmann bekannten allgemeinen Methoden der organischen Chemie. Dabei werden die Reaktionsbedingungen entsprechend der jeweiligen Umsetzung an die jeweilige Reaktion im Hinblick auf Reaktionsdauer, Temperatur, Druck und dergleichen so gewählt, dass eine maximale Produktausbeute der einzelnen Stufen erreicht wird. Reaktionsverläufe können beispielsweise durch Dünnschichtchromatographie überwacht werden.

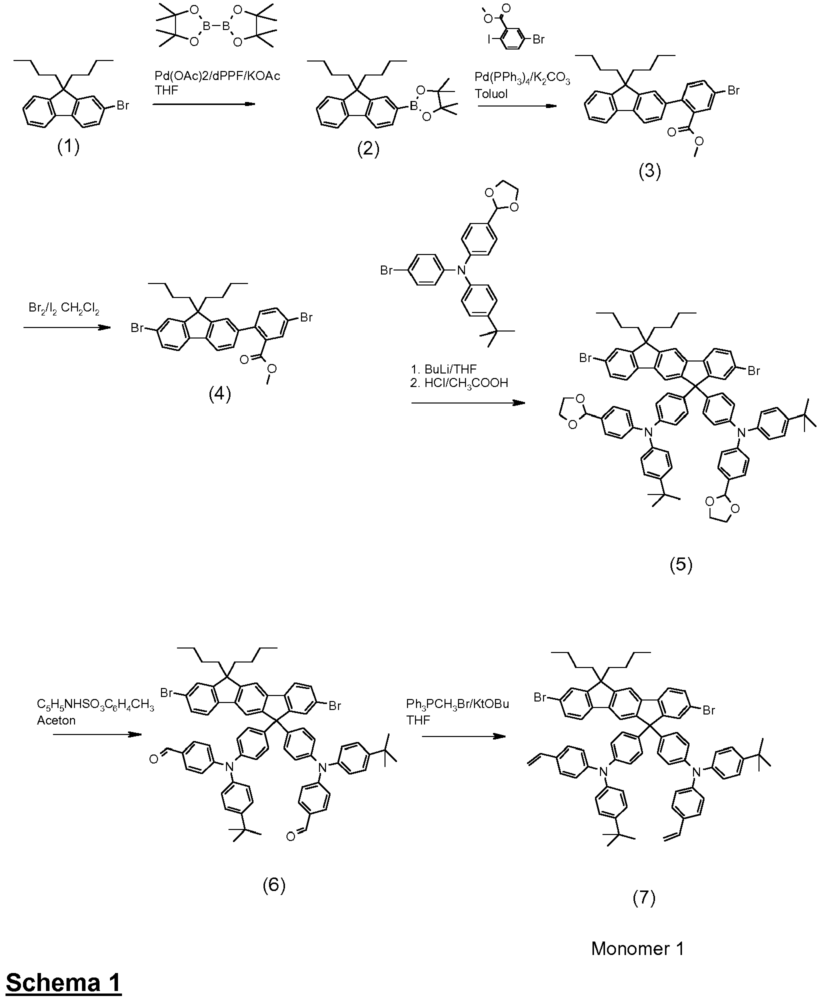

### Beispiel 2:

### Herstellung von Monomer 2:

Die Herstellung von Monomer 2 erfolgt gemäß dem unten dargestellten Schema 2. Die Umsetzungen in den einzelnen Stufen erfolgen dabei, wie unter Beispiel 1 genannt, nach dem Fachmann bekannten allgemeinen Methoden der organischen Chemie.

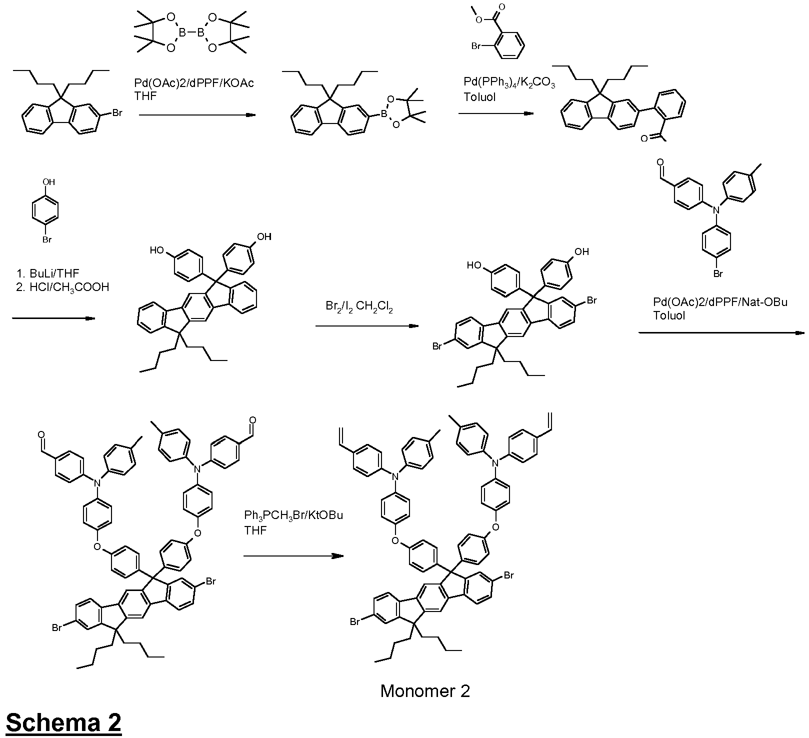

### B) Herstellung der Polymere

### Beispiel 3 bis 6:

### Herstellung der erfindungsgemäßen Polymere P2 und P3 sowie des Referenzpolymeren P1 sowie des Vergleichspolymers V1

Die erfindungsgemäßen Polymere P2 und P3 sowie das Referenzpolymer P1 sowie das Vergleichspolymer V1 werden durch SUZUKI-Kupplung gemäß WO 03/048225 synthetisiert. Die eingesetzten Monomerbausteine für die Synthese der Polymere **P1** bis **P3** sowie des Vergleichpolymers **V1** entsprechen den unten angegebenen Monomeren mit den dazu genannten prozentualen Anteilen, die in den entsprechenden Polymeren im wesentlichen unverändert wiederzufinden sind.

### Polymer P1:

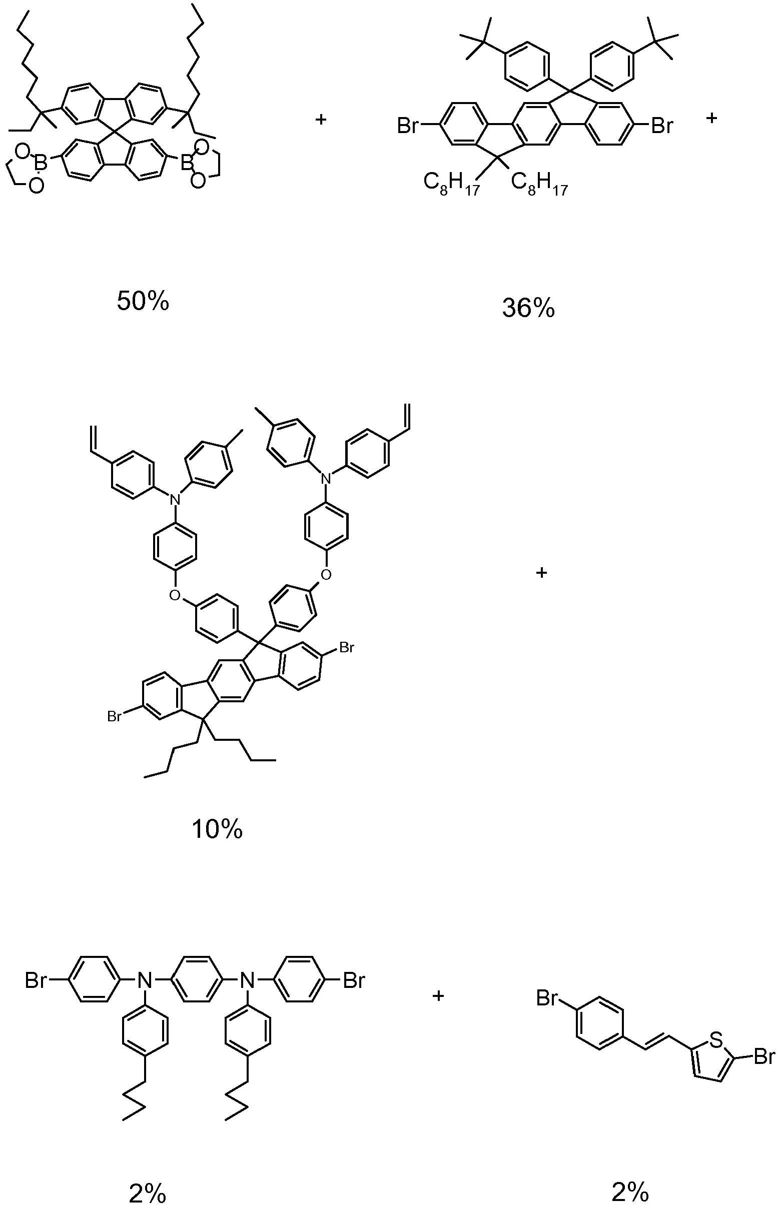

### Polymer P2:

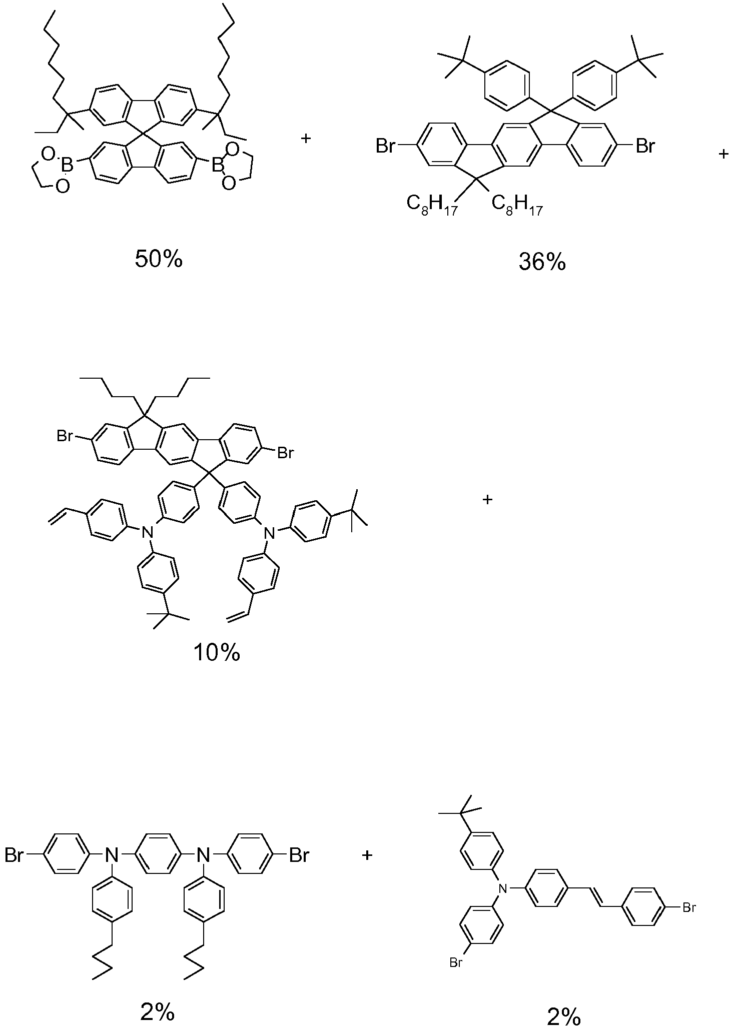

### Polymer P3:

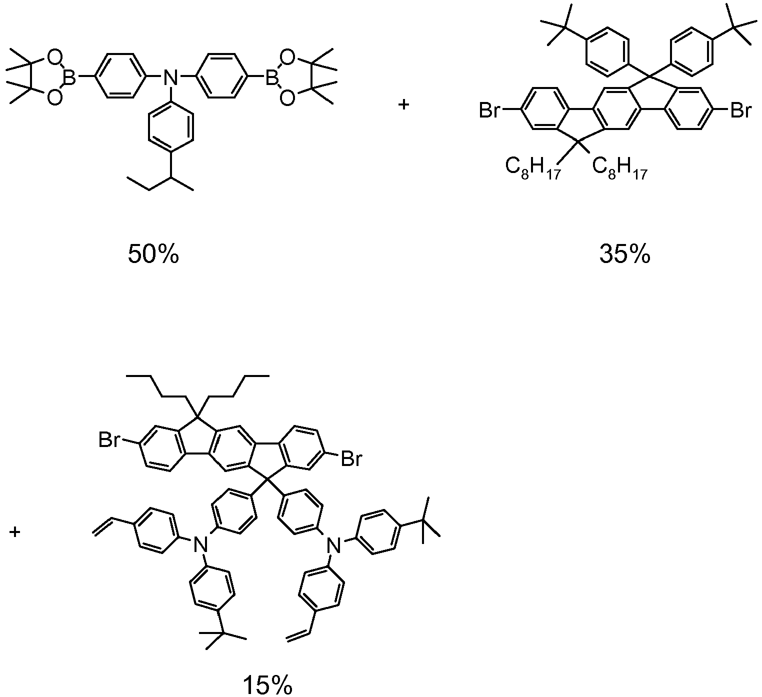

### Vergleichspolymer V1:

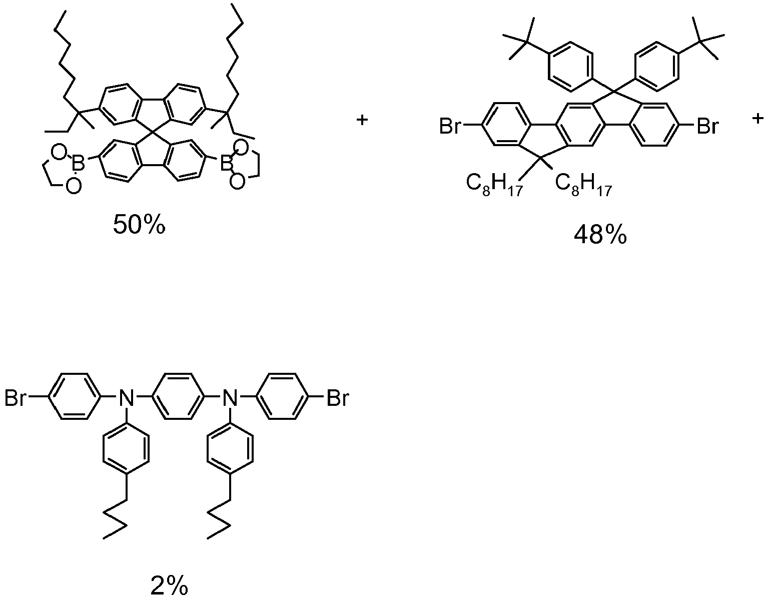

### C) Herstellung der polymeren Leuchtdioden (PLEDs)

### Beispiele 7 bis 10:

Die Herstellung einer polymeren Leuchtdiode (PLED) wird in der Patentliteratur bereits vielfach beschrieben (z.B. in der WO 04/037887). Um die vorliegende Erfindung beispielhaft zu erläutern, werden PLEDs mit den Polymeren P1 bis P3 sowie dem Vergleichspolymer V1 durch Spincoating auf zuvor mit PEDOT und einem lochinjizierenden Interlayer beschichtetem ITO-Substrat hergestellt. (PEDOT ist ein Polythiophen-Derivat (Baytron P, von H. C. Starck, Goslar)). Die Schichtdicke der Polymerschicht beträgt ca. 65 nm. Die Polymere P1 bis P3 werden nach dem Aufschleudern noch eine Stunde bei 180°C ausgeheizt, um das Polymer zu vernetzen. Danach wird eine Ba/Al-Kathode (Metalle von Aldrich) aufgedampft, die PLED verkapselt und elektrooptisch charakterisiert.

Die Ergebnisse, die bei Verwendung der Polymeren P1 bis P3 sowie V1 in PLEDs erhalten werden, sind in Tabelle 1 zusammengefasst.

Wie man aus den Ergebnissen erkennen kann, ist die Effizienz der erfindungsgemäßen Polymere besser als die des Vergleichspolymers. Die Lebensdauern sind deutlich verbessert. Dies zeigt, dass die erfindungsgemäßen Polymere besser für den Einsatz in OLEDs geeignet sind als Polymere gemäß dem Stand der Technik.

Für Polymer P3 werden keine Devicedaten genannt, da dieses als Zwischenschicht und nicht als Emitterschicht fungiert.

**Tabelle 1**

| Bsp. | Polymer | Max. Eff [Cd/A] | U@1000cd/m² [V] | CIE [x/y] | Lebensdauer [h] |
|---|---|---|---|---|---|
| 7 | P1 | 10,35 | 5,29 | 0,17/0,33 | 3000@1000 |
| 8 | P2 | 6,85 | 3,89 | 0,15/0,18 | 2200@1000 |
| 10 | V1 | 4,57 | 5,04 | 0,15/0,15 | 80@1000 |

### D) Kontrollversuche

### Überprüfung der Vernetzung der Polymeren

Auf die mit PEDOT und ITO beschichteten Substrate werden die Polymere P1 bis P3 aufgeschleudert. Die Schichtdicke wird gemessen. Der Polymerfilm wird eine Stunde bei 180°C ausgeheizt, um zu vernetzen. Dann wird mit Toluol "gewaschen" (auf spin-coater), der Film nochmals bei 180°C für 10 Minuten ausgeheizt und die Schichtdicke noch einmal gemessen, um zu überprüfen, ob Polymer heruntergewaschen wurde, das heißt, ob die Vernetzung erfolgreich war oder nicht.

Die Schichtdicke der erfindungsgemäßen Polymeren P2 und P3 sowie des Referenzpolymeren P1 (vor und nach der Vernetzung) sowie des Vergleichspolymers V1 sind in Tabelle 2 zusammengefasst.

**Tabelle 2**

| Polymer | Schichtdicke vor Vernetzung und Waschprozess [nm] | Schichtdicke nach Vernetzung und Waschprozess [nm] | Verhältnis vor/nach [%] |
|---|---|---|---|
| P1 | 67 | 63 | 94 |
| P2 | 65 | 62 | 95 |
| P3 | 66 | 65 | 98 |
| V1 | 65 | 23 | 35 |

Die Ergebnisse zeigen, dass das Vernetzen bei den Polymeren P1 bis P3 fast vollständig ist. Im Vergleich zu dem Vergleichspolymer V1, kann man mit den erfindungsgemäßen, vernetzbaren Polymeren die Schichtdicke kontrollieren.

## Patentansprüche

1. Polymer enthaltend 0,1 bis 50 mol% mindestens einer Struktureinheit der allgemeinen Formel I, 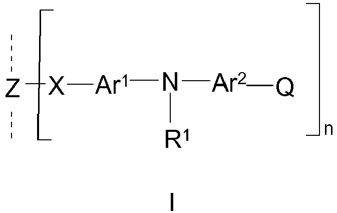 wobei
Z ein aromatisches oder heteroaromatisches Ringsystem mit 5 bis 60 Ringatomen ist, ausgewählt aus Indenofluoren, welches mit einem oder mehreren Resten R substituiert sein kann,
X ausgewählt ist aus einer direkten Bindung, einer linearen oder verzweigten Alkylen-Gruppe mit 1 bis 20 C-Atomen, in der eine oder mehrere nicht benachbarte CH₂-Gruppen durch -O-, -S-, -NH-, -N(CH₃)-, -N-CO-, -N-CO-O-, -N-CO-N, -CO-, -O-CO-, -S-CO-, -O-COO-, -CO-S-, -CO-O-, -CH(Halogen)-, -CH(CN)-, -CH=CH- oder -C≡C- ersetzt sein können, oder einer cyclischen Alkylgruppe,
Ar¹, Ar² jeweils unabhängig voneinander eine Aryl- oder Heteroarylgruppe mit 5 bis 60 Ringatomen sind, welche mit einem oder mehreren beliebigen Resten R substituiert oder miteinander verknüpft sein können, wobei Ar¹ eine 1,4-verknüpfte Aryl- oder Heteroarylgruppe ist,
Q eine vernetzbare Gruppe ist, ausgewählt aus Vinyl- oder Alkenylgruppe,
R bei jedem Auftreten unabhängig voneinander ausgewählt ist aus F, Cl, Br, I, N(Ar)₂, N(R²)₂, CN, NO₂, Si(R²)₃, B(OR²)₂, C(=O)Ar, C(=O)R², P(=O)(Ar)₂, P(=O)(R²)₂, S(=O)Ar, S(=O)R², S(=O)₂Ar, S(=O)₂R², -CR²=CR²Ar, OSO₂R², einer geradkettigen Alkyl-, Alkoxy- oder Thioalkoxygruppe mit 1 bis 40 C-Atomen oder einer verzweigten oder cyclischen Alkyl-, Alkoxy- oder Thioalkoxygruppe mit 3 bis 40 C-Atomen, die jeweils mit einem oder mehreren Resten R² substituiert sein kann, wobei eine oder mehrere nicht benachbarte CH₂-Gruppen durch R²C=CR², C≡C, Si(R²)₂, Ge(R²)₂, Sn(R²)₂, C=O, C=S, C=Se, C=NR², P(=O)(R²), SO, SO₂, NR², O, S oder CONR² ersetzt sein können und wobei ein oder mehrere H-Atome durch F, Cl, Br, I, CN oder NO₂ ersetzt sein können, oder einem aromatischen oder heteroaromatischen Ringsystem mit 5 bis 60 aromatischen Ringatomen, das jeweils durch einen oder mehrere Reste R² substituiert sein kann, oder einer Aryloxy- oder Heteroaryloxygruppe mit 5 bis 60 aromatischen Ringatomen, die durch einen oder mehrere Reste R² substituiert sein kann, oder einer Kombination dieser Systeme, wobei zwei oder mehrere Substituenten R auch miteinander ein mono- oder polycyclisches, aliphatisches oder aromatisches Ringsystem bilden können, wobei R² jeweils unabhängig voneinander H oder ein aliphatischer oder aromatischer Kohlenwasserstoffrest mit 1 bis 20 C-Atomen ist,
R¹ eine geradkettige Alkyl-, Alkoxy- oder Thioalkoxygruppe mit 1 bis 40 C-Atomen oder eine verzweigte oder cyclische Alkyl-, Alkoxy- oder Thioalkoxygruppe mit 3 bis 40 C-Atomen ist oder eine Silylgruppe oder eine substituierte Ketogruppe mit 1 bis 40 C-Atomen, oder ein substituiertes oder unsubstituiertes aromatisches oder heteroaromatisches Ringsystem mit 5 bis 60 Ringatomen, oder eine Aryloxy- oder Heteroaryloxygruppe mit 5 bis 60 Ringatomen, oder eine Kombination dieser Systeme, oder Ar² ist; wobei ein oder mehrere H-Atome durch Fluor ersetzt sein können,
n 1, 2, 3 oder 4 ist, und
die gestrichelten Linien die Verknüpfung im Polymer darstellen,
wobei gilt, wenn n = 1 ist,
dass X keine direkte Bindung ist.

2. Polymer nach Anspruch 1, **dadurch gekennzeichnet, dass** das Polymer weitere, von der allgemeinen Formel I verschiedene Struktureinheiten enthält.

3. Formulierung enthaltend ein Polymer nach Anspruch 1 oder 2 in einem oder mehreren Lösungsmitteln.

4. Elektronische Vorrichtung enthaltend ein Polymer nach Anspruch 1 oder 2.

5. Elektronische Vorrichtung nach Anspruch 4, **dadurch gekennzeichnet, dass** das Polymer vernetzt ist.

6. Elektronische Vorrichtung nach Anspruch 4 oder 5, **dadurch gekennzeichnet, dass** das Polymer in der Vorrichtung als Lochtransport-, Lochinjektions-, Emitter-, Elektronentransport-, Elektroneninjektions-, Ladungsblockier- und/oder Ladungserzeugungsschicht vorliegt.

7. Elektronische Vorrichtung nach einem oder mehreren der Ansprüche 4 bis 6, **dadurch gekennzeichnet, dass** die elektronische Vorrichtung eine organische elektrolumineszierende Vorrichtung (OLED), eine polymere elektrolumineszierende Vorrichtung (PLED), eine organische integrierte Schaltung (O-IC), ein organischer Feld-Effekt-Transistor (O-FET), ein organischer Dünnfilmtransistor (O-TFT), ein organischer lichtemittierender Transistor (O-LET), eine organische Solarzelle (O-SC), ein organischer optischer Detektor, ein organischer Photorezeptor, ein organisches Feld-Quench-Device (O-FQD), eine lichtemittierende elektrochemische Zelle (LEC) oder eine organische Laserdiode (O-Laser) ist.

## Claims

1. Polymer containing 0.1 to 50 mol% of at least one structural unit of the general formula I, 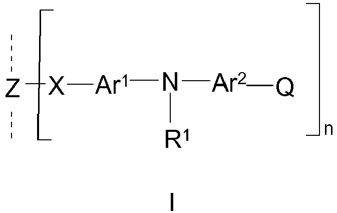 where
Z is an aromatic or heteroaromatic ring system having 5 to 60 ring atoms, selected from indenofluorene, which may be substituted by one or more radicals R,
X is selected from a direct bond, a linear or branched alkylene group having 1 to 20 C atoms, in which one or more non-adjacent CH₂ groups may be replaced by -O-, -S-, -NH-, -N(CH₃)-, -N-CO-, -N-CO-O-, -N-CO-N, -CO-, -O-CO-, -S-CO-, -O-COO-, -CO-S-, -CO-O-, -CH(halogen)-, -CH(CN)-, -CH=CH- or -C≡C-, or a cyclic alkyl group,
Ar¹, Ar² are each, independently of one another, an aryl or heteroaryl group having 5 to 60 ring atoms, which may be substituted by one or more radicals R of any desired type or may be linked to one another, where Ar¹ is a 1,4-linked aryl or heteroaryl group,
Q is a crosslinkable group selected from a vinyl or alkenyl group,
R is selected on each occurrence, independently of one another, from F, Cl, Br, I, N(Ar)₂, N(R²)₂, CN, NO₂, Si(R²)₃, B(OR²)₂, C(=O)Ar, C(=O)R², P(=O)(Ar)₂, P(=O)(R²)₂, S(=O)Ar, S(=O)R², S(=O)₂Ar, S(=O)₂R², -CR²=CR²Ar, OSO₂R², a straight-chain alkyl, alkoxy or thioalkoxy group having 1 to 40 C atoms or a branched or cyclic alkyl, alkoxy or thioalkoxy group having 3 to 40 C atoms, which may in each case be substituted by one or more radicals R², where one or more non-adjacent CH₂ groups may be replaced by R²C=CR², C≡C, Si(R²)₂, Ge(R²)₂, Sn(R²)₂, C=O, C=S, C=Se, C=NR², P(=O)(R²), SO, SO₂, NR², O, S or CONR² and where one or more H atoms may be replaced by F, Cl, Br, I, CN or NO₂, or an aromatic or heteroaromatic ring system having 5 to 60 aromatic ring atoms, which may in each case be substituted by one or more radicals R², or an aryloxy or heteroaryloxy group having 5 to 60 aromatic ring atoms, which may be substituted by one or more radicals R², or a combination of these systems, where two or more substituents R may also form a mono- or polycyclic, aliphatic or aromatic ring system with one another, where R² is in each case, independently of one another, H or an aliphatic or aromatic hydrocarbon radical having 1 to 20 C atoms,
R¹ is a straight-chain alkyl, alkoxy or thioalkoxy group having 1 to 40 C atoms or a branched or cyclic alkyl, alkoxy or thioalkoxy group having 3 to 40 C atoms or a silyl group or substituted keto group having 1 to 40 C atoms, or a substituted or unsubstituted aromatic or heteroaromatic ring system having 5 to 60 ring atoms, or an aryloxy or heteroaryloxy group having 5 to 60 ring atoms, or a combination of these systems, or Ar², where one or more H atoms may be replaced by fluorine,
n is 1, 2, 3 or 4, and
the dashed lines represent the linking in the polymer,
where, if n = 1,
X is not a direct bond.

2. Polymer according to Claim 1, **characterised in that** the polymer contains further structural units which are different from the general formula I.

3. Formulation comprising a polymer according to Claim 1 or 2 in one or more solvents.

4. Electronic device containing a polymer according to according to Claim 1 or 2.

5. Electronic device according to Claim 4, **characterised in that** the polymer is crosslinked.

6. Electronic device according to Claim 4 or 5, **characterised in that** the polymer is present in the device as hole-transport, hole-injection, emitter, electron-transport, electron-injection, charge-blocking and/or charge-generation layer.

7. Electronic device according to one or more of Claims 4 to 6, **characterised in that** the electronic device is an organic electroluminescent device (OLED), a polymeric electroluminescent device (PLED), an organic integrated circuit (O-IC), an organic field-effect transistor (O-FET), an organic thin-film transistor (O-TFT), an organic light-emitting transistor (O-LET), an organic solar cell (O-SC), an organic optical detector, an organic photoreceptor, an organic field-quench device (O-FQD), a light-emitting electrochemical cell (LEC) or an organic laser diode (O-laser).

## Revendications

1. Polymère contenant de 0,1 à 50 % (pourcentage molaire) d'au moins une unité structurelle de la formule générale I : 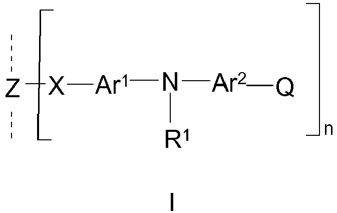 où
Z est un système de cycle aromatique ou hétéroaromatique qui comporte de 5 à 60 atomes de cycle, lequel est sélectionné parmi indénofluorène, lequel peut être substitué par un radical ou par plusieurs radicaux R ;
X est sélectionné parmi une liaison directe, un groupe alkylène linéaire ou ramifié qui comporte de 1 à 20 atome(s) de C, où un ou plusieurs groupe(s) CH₂ non adjacents peut/peuvent être remplacé(s) par -O-, -S-, -NH-, -N(CH₃)-, -N-CO-, -N-CO-O-, -N-CO-N, -CO-, -O-CO-, -S-CO-, -O-COO-, -CO-S-, -CO-O-, -CH(halogène)-, -CH(CN)-, -CH=CH- ou -C≡C-, ou un groupe alkyle cyclique ;
Ar¹, Ar² sont chacun, de manière indépendante l'un de l'autre, un groupe aryle ou hétéroaryle qui comporte de 5 à 60 atomes de cycle, lequel peut être substitué par un radical ou par plusieurs radicaux R de n'importe quel type souhaité, ou peuvent être liés l'un à l'autre, où Ar¹ est un groupe aryle ou hétéroaryle 1,4-lié ;
Q est un groupe réticulable qui est sélectionné parmi un groupe vinyle ou alkényle ;
R est sélectionné pour chaque occurrence, de manière indépendante les unes des autres, parmi F, Cl, Br, I, N(Ar)₂, N(R²)₂, CN, NO₂, Si(R²)₃, B(OR²)₂, C(=O)Ar, C(=O)R², P(=O)(Ar)₂, P(=O)(R²)₂, S(=O)Ar, S(=O)R², S(=O)₂Ar, S(=O)₂R², -CR²=CR²Ar, OSO₂R², un groupe alkyle, alcoxy ou thioalcoxy en chaîne droite qui comporte de 1 à 40 atome(s) de C ou un groupe alkyle, alcoxy ou thioalcoxy ramifié ou cyclique qui comporte de 3 à 40 atomes de C, lequel peut dans chaque cas être substitué par un radical ou par plusieurs radicaux R², où un ou plusieurs groupes CH2 non adjacents peut/peuvent être remplacé(s) par R²C=CR², C≡C, Si(R²)₂, Ge(R²)₂, Sn(R²)₂, C=O, C=S, C=Se, C=NR², P(=O)(R²), SO, SO₂, NR², O, S ou CONR² et où un ou plusieurs atome(s) de H peut/peuvent être remplacé(s) par F, Cl, Br, I, CN ou NO₂, ou un système de cycle aromatique ou hétéroaromatique qui comporte de 5 à 60 atomes de cycle aromatique, lequel peut dans chaque cas être substitué par un radical ou par plusieurs radicaux R², ou un groupe aryloxy ou hétéroaryloxy qui comporte de 5 à 60 atomes de cycle aromatique, lequel peut être substitué par un radical ou par plusieurs radicaux R², ou une combinaison de ces systèmes, où deux substituants R ou plus peuvent également former un système de cycle aliphatique aromatique mono- ou polycyclique l'un avec l'autre ou les uns avec les autres, où R² est dans chaque cas, de manière indépendante les uns des autres, H ou un radical hydrocarbone aliphatique ou aromatique qui comporte de 1 à 20 atome(s) de C ;
R¹ est un groupe alkyle, alcoxy ou thioalcoxy en chaîne droite qui comporte de 1 à 40 atome(s) de C ou un groupe alkyle, alcoxy ou thioalcoxy ramifié ou cyclique qui comporte de 3 à 40 atomes C ou un groupe silyle ou un groupe céto substitué qui comporte de 1 à 40 atome(s) de C, où un système de cycle aromatique ou hétéroaromatique substitué ou non substitué qui comporte de 5 à 60 atomes de cycle, ou un groupe aryloxy ou hétéroaryloxy qui comporte de 5 à 60 atomes de cycle, ou une combinaison de ces systèmes, ou Ar², où un ou plusieurs atome(s) de H peut/peuvent être remplacé(s) par fluor ;
n est 1, 2, 3 ou 4 ; et
les lignes en pointillés représentent la liaison dans le polymère ;
où, si n = 1,
X n'est pas une liaison directe.

2. Polymère selon la revendication 1, **caractérisé en ce que** le polymère contient d'autres unités structurelles qui sont différentes de la formule générale I.

3. Formulation comprenant un polymère selon la revendication 1 ou 2 dans un ou plusieurs solvant(s).

4. Dispositif électronique contenant un polymère selon la revendication 1 ou 2.

5. Dispositif électronique selon la revendication 4, **caractérisé en ce que** le polymère est réticulé.

6. Dispositif électronique selon la revendication 4 ou 5, **caractérisé en ce que** le polymère est présent dans le dispositif en tant que couche de transport de trous, d'injection de trous, d'émetteur, de transport d'électrons, d'injection d'électrons, de blocage de charges et/ou de génération de charges.

7. Dispositif électronique selon une ou plusieurs des revendications 4 à 6, **caractérisé en ce que** le dispositif électronique est un dispositif électroluminescent organique (OLED), un dispositif électroluminescent polymérique (PLED), un circuit intégré organique (O-IC), un transistor à effet de champ organique (O-FET), un transistor à film mince organique (O-TFT), un transistor à émission de lumière organique (O-LET), une cellule solaire organique (O-SC), un détecteur optique organique, un photorécepteur organique, un dispositif à extinction de champ organique (O-FQD), une cellule électrochimique à émission de lumière (LEC) ou une diode laser organique (O-laser).
